# EUROPÄISCHE PATENTANMELDUNG

(11) **EP 3 766 897 A1**
(43) Veröffentlichungstag der Anmeldung: **20.01.2021**
(21) Anmeldenummer: 19186656.5
(22) Anmeldetag: 16.07.2019
(51) Int. Cl.: C07K 14/71, A63F 7/02, G06Q 50/00

(54) **VERFAHREN ZUR SELEKTION VON HYBRIDOMZELLEN AUS EINER VIELZAHL VON HYBRIDOMZELLEN MITTELS EINES BIRA-EXPRESSIONSVEKTORS**

(71) Anmelder: New/era/mabs GmbH, 14482 Potsdam (DE)
(72) Erfinder: HANACK, Katja, 14169 Berlin (DE); LISTEK, Martin, 13059 Berlin (DE); MICHEEL, Burkhard, 17268 Gerswalde (DE)
(74) Vertreter: Simandi, Claus

(57) **Zusammenfassung**

Die Erfindung betrifft ein verbessertes Verfahren zur Selektion von Hybridomzellen aus einer Vielzahl von Hybridomzellen zur Gewinnung von monoklonalen Antikörpern als auch Hybridomzellen samt geeigneten Expressionsvektoren mithilfe einer intrazellulären Biotinylierung und deren Verwendung.

## Beschreibung

Die Erfindung betrifft ein verbessertes Verfahren zur Selektion von Hybridomzellen aus einer Vielzahl von Hybridomzellen zur Gewinnung von monoklonalen Antikörpern als auch Hybridomzellen samt geeigneten Expressionsvektoren und deren Verwendung.

Monoklonale Antikörper werden in der Regel mit Hilfe der Hybridomtechnik gewonnen, wobei antikörperproduzierende Zellen (B-Zellen oder B-Lymphozyten) mit Myelomzellen (Krebszellen) fusionieren, auch mittels Fusionszelllinien, woraufhin Hybride entstehen, die monoklonale Antikörper produzieren (G. Köhler, C. Milstein: Continuous cultures of fused cells secreting antibody of predefined specificity. In: Nature. Band 256, 1975, S. 495-497). Dabei gestaltet sich nach wie vor trotz Modifizierungen und Weiterentwicklungen der Hybridomtechnik die Identifizierung und Etablierung der gewünschten antigenspezifischen Hybridomzellen innerhalb des sehr heterogenen Zellpools bis heute als schwierig und zeitaufwendig.

Im Stand der Technik und zwar WO2015161835 A1 der Anmelderin ist die Selektion von Antikörper-produzierenden Hybridomzellen beschrieben, wobei die von der produzierenden Zelle sezernierten Antikörper an deren Zelloberfläche gebunden werden, um die in der Weise oberflächenmarkierten Zellen mittels fluoreszenzmarkierter Nachweis-Antikörper zu identifizieren und zu isolieren und zwecks Gewinnung selektiv zu vermehren. Hierbei werden durch die Verwendung künstlicher Oberflächenmarker die Antikörper-produzierenden Hybridomzellen modifiziert. Dadurch entsteht eine direkte Verbindung zwischen dem Antikörperphänotyp und dem Genotyp der Hybridomzelle. Auf diese Weise können Hybridomzellen, die die gewünschten Antikörper produzieren, aus einem heterogenen Zellpool insbesondere mittels MACS- oder FACS-Technik äußerst effizient, sowie zeitsparend angereichert werden.

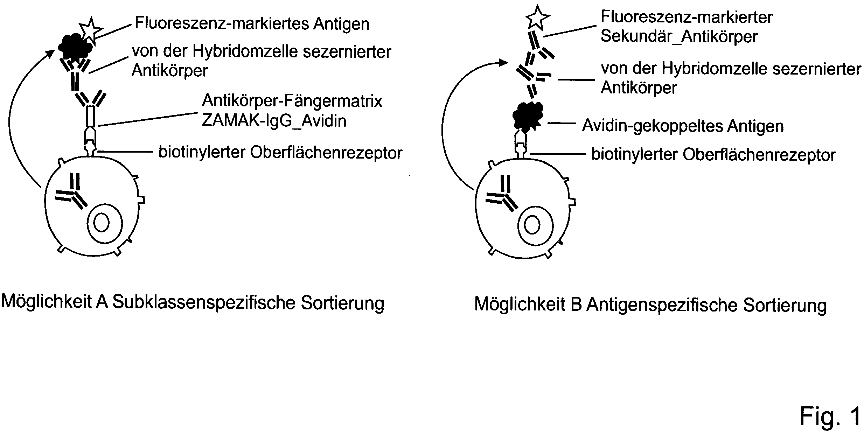

WO2015161835 A1 beschreibt daher den Einsatz einer Ligationspeptidsequenz, mit der Funktion, dass die Bindung eines Adapterliganden erfolgen kann, nämlich vorzugsweise Biotin, dass unter Zugabe aus Vollmedium unter schonenden Bedingungen mittels einer aus E. coli gewonnenen BirA (Biotin-Proteinligase) -Ligase mit der Ligationspeptidsequenz (bzw. Biotinylierungspeptid) im Verhältnis 1:1 enzymatisch konjugiert wird und infolgedessen eine Biotinylierung an der Ligationspeptidsequenz erfolgt. An Biotin erfolgt die Bindung des freigesetzten Antikörpers aus der Hybridomzelle über einen Epitop aufweisenden Antigen-(Strept)avidin-Komplex, wobei der Antikörper an das Epitop des Antigens bindet und über die Streptavidin-Biotin Bindung, wiederum der Hybridomzelle zugeordnet und (aus)sortiert werden kann, sei es zum Beispiel mittels einer Antikörper-Fängermatrix oder einem Antigen, welches z.B. über (Strept)Avidin gekoppelt werden kann (siehe Figur 1).

Mit Hilfe der Durchflusszytometrie können beispielsweise solche Fluoreszenz-markierte Konstrukte gemäß Figur 1A (Antikörper-Fängermatrix) oder Figur 1B (Antigen) mit einem flow sorter (Fluss-Sortierer) oder FACS (fluorescence-activated cell sorting) sortiert und isoliert werden.

Die Ligationspeptidsequenz weist eine Biotinakzeptorpeptidsequenz bzw. Biotinylisierungspeptid, wie vorzugsweise GLNDIFEAQKIEWHE (SEQ ID No. 1) oder LNDIFEAQKIEWH (SEQ ID No. 2) auf. Auch andere Biotinakzeptorpeptidsequenz bzw. Biotinylisierungspeptide können geeignet sein, wie ein Alignment mit 13 Aminosäuren 1-**L**XX**I**XXXX**K**X XXX-13 gemäß SEQ ID. No. 3 (vgl. D. Beckett, E. Kovaleva, and P. J. Schatz, A minimal peptide substrate in biotin holoenzyme synthetase-catalyzed biotinylation, Protein Sci. 1999 Apr; 8(4): 921-929), wobei:
Aminosäure 2 N, C oder beliebig oder entfernt sein kann,
Aminosäure 3 beliebig, jedoch ausgenommen L, V, I, W, F, Y sein kann,
Aminosäure 5 F oder L sein kann,
Aminosäure 6 E oder D sein kann,
Aminosäure 7 A, G, S, T sein kann,
Aminosäure 8 Q oder M sein kann,
Aminosäure 10 I, M, V sein kann,
Aminosäure 11 E, L, V, Y, I sein kann,
Aminosäure 12 W, Y, V, F, L, I sein kann,
Aminosäure 13 R, H oder beliebig, jedoch ausgenommen D, E.

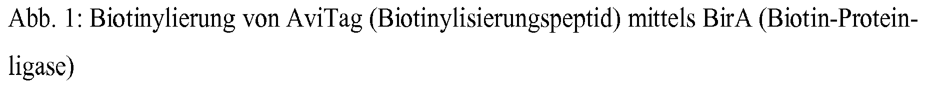

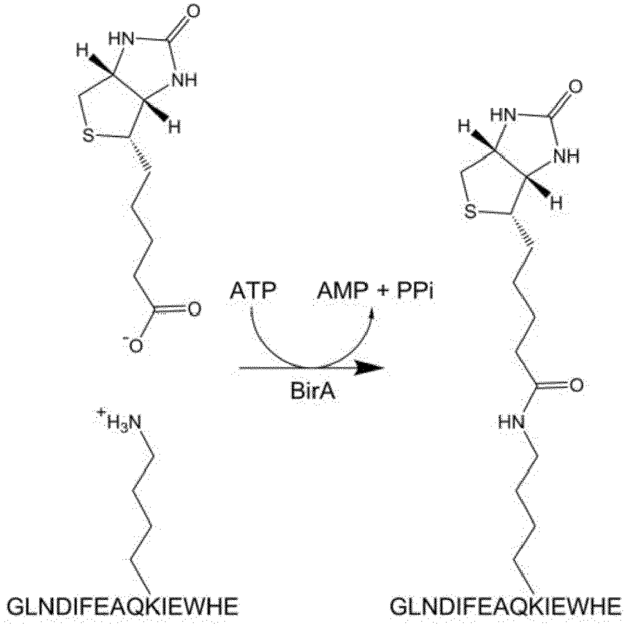

Nachteilig an diesem Verfahren ist jedoch, dass die Biotin-Proteinligase extern hinzugegeben werden muss und oftmals die Menge nicht ausreichend ist, so dass eine Optimierung und Anpassung für jeden neuen Ansatz erforderlich sind.

Die Biotin-Proteinligase (=BirA, SEQ ID No. 4) ist im Stand der Technik ausführlich beschrieben (O'Callaghan et al, BirA Enzyme: Production and Application in the Study of Membrane Receptor-Ligand Interactions by Site-Specific Biotinylation, Analytical Biochemistry 266, 9-15 (1999)) und DE69434520T2 (Schatz) beschreibt ein Verfahren zur Herstellung von biotinylierten Proteinen in vitro und in rekombinanten Wirtszellen unter Verwendung von Expressionsvektoren, welche für ein Fusionsprotein kodieren, nämlich ein Protein aufweisend ein Biotinylierungspeptid.

Durch Kultivieren der Wirtszelle in Gegenwart von Biotin, kann das Fusionsprotein und das Biotinylierungsenzym exprimiert werden, wodurch eine Biotinylierung des Fusionsproteins erfolgt.

DE69434520T2 (Schatz) offenbart jedoch nicht die Biotinylierung in Fusionspeptiden von Hybridomzellen.

In vivo wird Biotin an Proteinen hinzugefügt durch die Bildung einer Amidbindung zwischen der Biotin- Carboxylgruppe und der Epsilon-Aminogruppe von spezifischen Lysinresten in einer biochemischen Reaktion, die ATP benötigt (Zwischenprodukt ist Biotinoyl-AMP). In E. coli ist ein Protein biotinyliert, nämlich die Biotincarboxylcarrierprotein (kurz: BCCP) -Untereinheit der Acetyl-CoA-Carboxylase. Diese Reaktion wird katalysiert durch die Biotin-Proteinligase (BirA), das Produkt des birA-Gens, siehe Abb. 1 (supra).

Im Stand der Technik sind Hybridomzellen sehr heterogene Zellpools, welche zu anderen Wirtsorganismen in der Expression von rekombinanten Polynukleotiden deutlich verschieden sind. Hybridomzellen entstehen aus der Fusion von B-Lymphozyten mit Myelomzellen. Dieser Vorgang führt zum Verschmelzen zweier Genome. Die aus der Fusion entstehenden Hybridome weisen daher zunächst vier Chromosomensätze auf, die anschließend auf zwei Chromosomen reduziert werden. Dabei werden Gene nach einem Zufallsprinzip von den Zellen deletiert, was den gesamten Prozess sehr instabil und nicht steuerbar macht. Dies steht im Gegensatz zu herkömmlichen Säugerzell-Expressionssystemen wie CHO oder HEK293 (Schatz, supra).

Somit ist der nach der Fusion erhaltene Zellpool extrem heterogen und die spezifisch produzierenden Zellen müssen schnell identifiziert werden, da sie andernfalls von Nicht-Produzenten überwachsen werden. Die Fusionen und der Erhalt an spezifischen Zellen können aufgrund des instabilen Prozesses nicht validiert oder standardisiert werden.

Daher ist jede Fusion ein neues einzelnes Ereignis. Die Sortierung über einen extern eingebrachten Oberflächenmarker als stabiles Element würde eine Standardisierung zulassen. Jedoch ist die Selbstbiotinylierung zwingend erforderlich, da nicht vorher für jede Fusion die optimalen Biotinylierungsbedingungen ermittelt werden können.

Daher ist es Aufgabe der Erfindung, das bekannte Verfahren aus WO2015/161835 A1 weiter zu verbessern. Insbesondere soll der Einsatz der Biotin-Proteinligase (BirA) in das Selektionsverfahren integriert werden als auch die erforderliche Biotinylierung eines Biotinylierungspeptid standardisiert werden.

Überraschender Weise konnte nunmehr gezeigt werden, dass mittels Expressionsvektoren, welche für Biotin-Proteinligase (BirA) kodieren, in Hybridomzellen eine intrazelluläre Biotinylierung eines (Fusions-)Proteins erfolgen kann, insbesondere in Form eines Oberflächenproteins aufweisend ein Biotinylierungspeptid, welches Biotin präsentiert, so dass zum Beispiel eine Antikörper-Fängermatrix oder Antigen über z.B. (Strept)Avidin oder Äquivalente (Neutravidin u.v.a,) gekoppelt werden kann.

In einer bevorzugten Ausführungsform der Erfindung ist die Transkription des Oberflächenproteins aufweisend ein Biotinylierungspeptid und einer Biotin-Proteinligase (BirA) aus einem Expressionsvektor vorzugsweise durch mindestens einen Promotor kontrolliert. Beide Gene können vorzugsweise über übliche Hilfssequenzen, wie IRES verbunden sein.

Durch eine vorteilhafte in vivo Biotinylierung des Expressionsproduktes innerhalb der Hybridomzellen kann auf anfallende Wasch- und Zentrifugationsschritte sowie die Verwendung von speziellen Puffern verzichtet werden, die die Vitalität der Zellen nachhaltig beeinträchtigen.

Insbesondere erfolgt erfindungsgemäß die in vivo Biotinylierung unter natürlichen, physiologischen Bedingungen (z.B. Kulturmedium im Brutschrank) bei dem die Hybridomzellen im Kulturmedium bleiben. Die Vitalität der Zellen bleibt vorteilhaft unberührt. Das Kulturmedium kann Biotin enthalten.

Besonders vorteilhaft wird erfindungsgemäß Biotin-Proteinligase (BirA) intrazellulär freigesetzt. Die Biotinylierung eines Biotinylierungspeptid erfolgt intrazellulär vorzugsweise im endoplasmatischen Retikulum. Anschließend wird der biotinylierte Rezeptor bzw. das Biotin aufweisende Biotinylisierungspeptid an die Zelloberfläche transportiert, und zwar in Form eines Oberflächenproteins aufweisend ein Biotinylierungspeptid, welches Biotin bindet und präsentiert.

Die Erfindung betrifft daher eine Hybridomzelle mit mindestens einem nach seiner Transformation stabil in das Genom integrierten Polynukleotid (birA) kodierend für Biotin-Proteinligase (BirA), insbesondere mittels eines Expressionsvektor aufweisend Promotoren und ggfs. weitere Hilfssequenzen, solche wie Enhancer, Terminatoren, Spacer, Signalsequenzen u.a. zur kontrollierten Expression von Biotin-Proteinligase (BirA).

Daher betrifft die Erfindung eine Hybridomzelle enthaltend mindestens einem nach seiner Transformation stabil in das Genom integrierten Polynukleotid kodierend für Biotin-Proteinligase (BirA), vorzugweise zusätzlich kodierend für ein Oberflächenprotein enthaltend ein Biotinylierungspeptid.

In einer bevorzugten Ausführungsform enthält der erfindungsgemäße Expressionsvektor folgende Merkmale, wie:
Signalsequenz, HA-Tag (SEQ ID No. 5, SEQ ID No. 6), DNA-Biotinylierungssequenz (supra, SEQ ID No. 7), EGFR-Signalsequenz (z.B. SEQ ID No. 8, SEQ ID No. 9), EGFR-Sequenz (SEQ ID No. 10, SEQ ID No. 11 inkl. Transmenbrandomäne), IRES (SEQ ID No. 12) als auch birA-Sequenz (SEQ ID No. 13 inkl. Retentionssignal) bzw. BirA (SEQ ID No. 14 inkl. Retentionssignal). Ein beispielhaftes Gesamtkonstrukt eines Expressionsvektors ist in SEQ ID No. 15 wiedergegeben.

Die angegebenen Sequenzen umfassen Polynukleotide als auch die exprimierten und translatierten Sequenzen.

Bevorzugte Promotoren sind nicht abschließend T7, EF-1, CMV, Hühner-β-Aktin, u.v.a. auch konditionierbare Promotoren, wie TET on, off, lichtabhängige Promotoren oder stressinduzierbare Promotoren (z.B. Temperatur) als auch mit Hilfe der Verwendung von Hilfssequenzen, wie IRES (internal ribosome entry site) 2A Peptide u.a.. Solche Promotoren und Hilfssequenzen sind dem Fachmann einschlägig bekannt.

In einer weiteren bevorzugten Ausführungsform enthält BirA ein Retentionssignal für das endoplasmatische Retikulum, wie KDEL (SEQ ID No. 14).

Das erfindungsgemäße Oberflächenprotein kann beispielsweise mit Hilfe von einem Epidermal Growth Factor Receptor (EGFR (supra), ErbB-1, HER1 u.a.) sicher an die Zelloberfläche transportiert und an der Zelloberfläche repräsentiert werden.

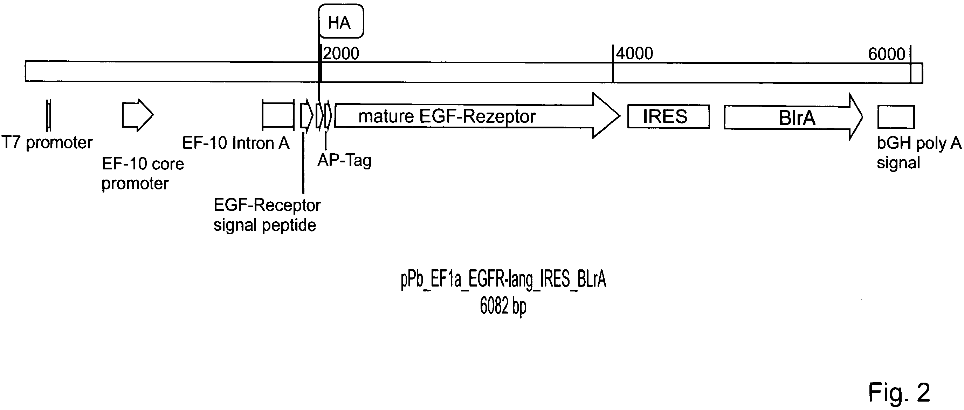

Ein erfindungsgemäßer Expressionsvektor ist in seinen funktionellen Einheiten (Vektorkarte) beispielhaft in Figur 2 wiedergegeben.

In einer weiteren Ausführungsform betrifft der Expressionsvektor daher einen PiggyBac-Vektor der Transposon Klasse entsprechend der Vektorkarte aus Figur 2 mit einem 5'ITR (Li X et al, Insect Mol Biol. 2005 Jan;14(1):17-3) und 3'ITR (Troyanovsky et al, Mol Ther Nucleic Acids. 2016 Oct 4;5(10):e369. doi: 10.1038/mtna.2016.76.) gemäß der SEQ ID No. 16 oder exprimiert und translatiert gemäß SEQ ID No. 17.

In einer weiteren Ausführungsform kann ein duales System bestehend aus einem Donor- und Helferplasmid verwendet werden, bei dem die Transposase auf einem Helferplasmid kodiert ist. Das Donorplasmid beinhaltet eine Kassette, die ins Genom integriert wird. Es besteht auch die Möglichkeit, dass die Transposase und das Donor-Konstrukt auf einem Vektor kodiert sind.

Ein beispielhafter Expressionsvektor gemäß der Vektorkarte aus Figur 2 ist für SEQ ID No. 18 mit 4175 bp angegeben und hinterlegt unter DSM 32960 am Leibniz-Institut DSMZ - Deutsche Sammlung von Mikroorganismen und Zellkulturen GmbH, Inhoffenstraße 7B, 38124 Braunschweig, Deutschland zum 14. November 2018 nach dem Budapest Treaty on the International Recognition of the Deposit of Microorganisms for the Purposes of Patent Procedure vom 28. April 1977.

In einer weiteren bevorzugten Ausführungsform der Erfindung ist eine solche Hybridomzelle enthaltend mindestens einem nach seiner Transformation stabil in das Genom integrierten Polynukleotid zumindest kodierend für Biotin-Proteinligase (BirA) mittels einer hinterlegten Fusionszelle hergestellt und zwar die Hinterlegungen DSM ACC 3343 oder DSM ACC 3344 am Leibniz-Institut DSMZ - Deutsche Sammlung von Mikroorganismen und Zellkulturen GmbH, Inhoffenstraße 7B, 38124 Braunschweig, Deutschland zum 14. November 2018 nach dem Budapest Treaty on the International Recognition of the Deposit of Microorganisms for the Purposes of Patent Procedure vom 28. April 1977.

Daher betrifft die Erfindung ebenfalls eine Fusionszelllinie DSM ACC 3343 oder DSM ACC 3344 enthaltend ein Polynukleotid kodierend für Biotin-Proteinligase (BirA) und für ein Oberflächenprotein enthaltend ein Biotinylierungspeptid, welche in einem Expressionsvektor enthaltend ist, und durch einen Promotor kontrolliert wird.

Weiterhin betrifft die Erfindung einen Kit aufweisend eine Fusionszelllinie DSM ACC 3343 und / oder DSM ACC 3344 enthaltend ein Polynukleotid kodierend für Biotin-Proteinligase (BirA) in einem Expressionsvektor.

Die Erfindung betrifft ebenfalls entsprechende Verfahren zur Herstellung der erfindungsgemäßen Hybridomzelle, insbesondere ein Verfahren zum Herstellen einer Hybridomzelle enthaltend mindestens einem nach seiner Transformation stabil in das Genom integrierten Polynukleotid kodierend für Biotin-Proteinligase (BirA), wobei das Polynukleotid kodierend für Biotin-Proteinligase (BirA) in einem Expressionsvektor (supra) verwendet wird. Weiterhin ein Verfahren zum Herstellen einer Hybridomzelle enthaltend mindestens einem nach seiner Transformation stabil in das Genom integrierten Polynukleotid kodierend für Biotin-Proteinligase (BirA) und für ein Oberflächenprotein enthaltend ein Biotinylierungspeptid, wobei das Polynukleotid kodierend für Biotin-Proteinligase (BirA) und für ein Oberflächenprotein enthaltend ein Biotinylierungspeptid in einem Expressionsvektor (supra) verwendet wird.

Ein weiterer Gegenstand betrifft ein Verfahren oder die Verwendung einer Hybridomzelle enthaltend mindestens einem nach seiner Transformation stabil in das Genom integrierten Polynukleotid kodierend für Biotin-Proteinligase (BirA) zur Durchführung einer Selektion von sezernierten monoklonalen Antikörpern, wobei eine Bindung der freigesetzten Antikörper aus dieser Hybridomzelle an das Oberflächenprotein aufweisend ein Biotinylierungspeptid erfolgt, umfassend eine enzymatische Konjugation von Biotin mittels Biotin-Proteinligase (BirA) mit einem Biotinylierungspeptid (Ligationspeptidsequenz), und ein Antigen oder eine Antikörper-Fängermatrix aufweisend ein Epitop für den freigesetzten Antikörper und jener Antikörper detektierbar ist.

Der aus der Hybridomzelle sezernierte Antikörper kann daher vorteilhaft mittels einer Antikörper-Fängermatrix oder einem Antigen über z.B. (Srept)Avidin an dem Biotinylierungspeptid aufweisend Biotin gebunden werden (siehe Figur 1A oder 1B).

Die folgenden Beispiele und Figuren sollen die Erfindung näher erläutern, ohne jedoch diese zu beschränken.

### Beispiel 1:

Beschreibung der Herstellung der Hybridomzelle in Gegenwart des Antigens (Beziehung Hybridom/Antigen), so dass das Antigen später im Konstrukt mit (Strept)avidin repräsentiert wird:
Die Herstellung der Hybridomzellen erfolgt zum Protokoll, veröffentlicht in: Holzlöhner P, Hanack K (2017) "Generation of Murine Monoclonal Antibodies by Hybridoma Technology." J Vis Exp. Jan 2;(119). doi: 10.3791/54832.

Nach der erfolgreichen Immunisierung mit dem Antigen werden der Maus die Milzzellen entnommen und diese mit Myelomzellen zu Hybridomen fusioniert. Hierzu werden die hinterlegten Fusionszelllinien (supra) eingesetzt. Vor der Fusion erfolgt eine Qualitätskontrolle, ob die veränderten Zelllinien das Oberflächenkonstrukt aufweisen. Hierfür wird folgendes Protokoll verwendet:
Für die Überprüfung des Vorhandenseins des Oberflächenkonstrukts werden die transgenen Myelomzellen (5x10⁶) geerntet, mit PBS gewaschen und mit einem murinen anti-HA-Antikörper (1 µg auf 1x10⁶ Zellen) für 30 min bei 4°C inkubiert. Danach werden die Zellen erneut zweimal gewaschen und mit 10 µL einer anti-murinen IgG Microbead Lösung für 15 min bei 4°C inkubiert. Nach einem zweimaligen Waschschritt werden die Zellen über eine magnetische Säule sortiert. Das erhaltene Zellpellet wird in Vollmedium aufgenommen. Die Zellen werden 2 Tage in dem Vollmedium kultiviert. Dieses enthält natürlicherweise Biotin, so dass die Zellen während dieser Inkubation gleichmäßig und vollständig biotinyliert werden. Die Überprüfung der erfolgreichen Biotinylierung erfolgt über die Zugabe von PE-markiertem Streptavidin und einer Inkubation von 20 min bei 4°C. Nach Bestätigung der erfolgreichen Biotinylierung der Zellen werden diese für die Fusion mit B-Lymphozyten eingesetzt.

Nach der Fusion erfolgt die herkömmliche HAT-Selektion. Danach werden die Zellen auf normales Vollmedium gesetzt.

Nach 10 Tagen HAT Selektion werden die Zellen auf Vollmedium gesetzt. Für die Sortierung erfolgt dann ein Ernten und Waschen der Zellen. Danach werden diese entweder mit der Antikörper-Fängermatrix (ZAMAK-IgG-Avidin) oder mit dem Avidin-gekoppelten Antigen (z.B. Ovalbumin) für 3 Stunden bei 37°C inkubiert. Nach einem erneuten Waschschritt erfolgt die Inkubation der Zellen entweder mit Fluoreszenz-markiertem Antigen oder mit einem Fluoreszenz-markierten Sekundär-Antikörper für 20 min bei 4°C. Die Zellen werden wieder gewaschen und das Pellet in 500 µL Puffer aufgenommen. Im Anschluss erfolgt die durchflusszytometrische Analyse und das Sorting der Zellen.

Möglichkeit A (Figur 1) ist die Herstellung einer Antikörperfängermatrix, bei der ein Subklassen-spezifischer Antikörper, in diesem Fall ein Ziege-anti-Maus IgG Antikörper (ZAMAK-IgG) an Avidin gekoppelt wird. Die Kopplung erfolgt nach einem Standardprotokoll. Dafür werden 1 mg des ZAMAK-IgG mit 0,5 mg Avidin und 0,25% Glutaraldehyd versetzt, mit 1x PBS auf 1 mL aufgefüllt und für 2 Stunden bei 4°C inkubiert. Anschließend erfolgt die Zugabe von Natriumborhydrid (Stammlösung 100 mg/mL) und einer weiteren Inkubation von 2 Stunden bei 4°C. Anschließend wird das Gemisch bei 13000 x g zentrifugiert und der Überstand gegen 1x PBS dialysiert. Das Kopplungskonjugat kann dann sterilfiltriert und gelagert werden.

Möglichkeit B (Figur 1) ist ein Ausführungsbeispiel für den Fall, dass das Antigen Ovalbumin ist. Der Ablauf der Kopplung erfolgt genauso, wie unter Möglichkeit A beschrieben. Für Ovalbumin werden jedoch 3 mg der Substanz mit 2 mg Avidin und 0,25% Glutaraldehyd versetzt, mit PBS auf 1 mL aufgefüllt und wie oben beschrieben weiter behandelt.

Danach wird mittels ELISA geprüft, ob die Matrix positiv ist, d.h. Antigen/Antikörper und Avidin vorhanden ist.

## Patentansprüche

1. Hybridomzelle enthaltend mindestens einem nach seiner Transformation stabil in das Genom integrierten Polynukleotid kodierend für eine Biotin-Proteinligase.

2. Hybridomzelle nach Anspruch 1, enthaltend mindestens einem nach seiner Transformation stabil in das Genom integrierten Polynukleotid kodierend für ein Oberflächenprotein enthaltend ein Biotinylierungspeptid.

3. Hybridomzelle nach Anspruch 1 oder Anspruch 2, aufweisend ein Oberflächenprotein enthaltend ein Biotinylierungspeptid, insbesondere ausgewählt aus der Gruppe SEQ ID No. 1, SEQ ID No. 2 oder SEQ ID No. 3.

4. Hybridomzelle nach einem der vorstehenden Ansprüche, **dadurch gekennzeichnet, dass** das integrierte Polynukleotid kodierend für Biotin-Proteinligase in einem Expressionsvektor enthaltend ist.

5. Hybridomzelle nach einem der vorstehenden Ansprüche, **dadurch gekennzeichnet, dass** das integrierte Polynukleotid kodierend für Biotin-Proteinligase und für ein Oberflächenprotein enthaltend ein Biotinylierungspeptid in einem Expressionsvektor enthaltend ist, und durch einen Promotor kontrolliert wird.

6. Hybridomzelle nach einem der vorstehenden Ansprüche, **dadurch gekennzeichnet, dass** Biotin-Proteinligase intrazellulär freigesetzt wird, und das Biotinylierungspeptid intrazellulär biotinyliert wird.

7. Hybridomzelle nach einem der vorstehenden Ansprüche, **dadurch gekennzeichnet, dass** das integrierte Polynukleotid kodierend für Biotin-Proteinligase in einem Expressionsvektor enthaltend ist, aufweisend zumindest eine Sequenz aus SEQ ID No. 7, SEQ ID No. 9 und SEQ ID No. 13 oder SEQ ID No. 15 oder SEQ ID No. 16 oder SEQ ID No. 18.

8. Hybridomzelle nach einem der vorstehenden Ansprüche, **dadurch gekennzeichnet, dass** eine Fusionszelllinie DSM ACC 3343 oder DSM ACC 3344 verwendet wird.

9. Verfahren zum Herstellen einer Hybridomzelle enthaltend mindestens einem nach seiner Transformation stabil in das Genom integrierten Polynukleotid kodierend für Biotin-Proteinligase, wobei das Polynukleotid kodierend für Biotin-Proteinligase in einem Expressionsvektor verwendet wird.

10. Verfahren zum Herstellen einer Hybridomzelle nach Anspruch 9 enthaltend mindestens einem nach seiner Transformation stabil in das Genom integrierten Polynukleotid kodierend für Biotin-Proteinligase und für ein Oberflächenprotein enthaltend ein Biotinylierungspeptid, wobei das Polynukleotid kodierend für Biotin-Proteinligase und für ein Oberflächenprotein enthaltend ein Biotinylierungspeptid in einem Expressionsvektor verwendet wird.

11. Verwendung einer Hybridomzelle enthaltend mindestens einem nach seiner Transformation stabil in das Genom integrierten Polynukleotid kodierend für Biotin-Proteinligase zur Durchführung einer Selektion von sezernierten monoklonalen Antikörpern.

12. Fusionszelllinie DSM ACC 3343 oder DSM ACC 3344.

13. Fusionszelllinie DSM ACC 3343 oder DSM ACC 3344 nach Anspruch 12 enthaltend ein Polynukleotid kodierend für Biotin-Proteinligase und für ein Oberflächenprotein enthaltend ein Biotinylierungspeptid, welche in einem Expressionsvektor enthaltend ist, und durch einen Promotor kontrolliert wird.

14. DSM 32960 enthaltend SEQ ID No. 18.

15. Kit enthaltend eine Fusionszelllinie DSM ACC 3343 und / oder DSM ACC 3344 nach Anspruch 12 enthaltend ein Polynukleotid kodierend für Biotin-Proteinligase in einem Expressionsvektor.


SEQUENCE LISTING

<110>  new/era/mabs GmbH

<120>  Verfahren zur Selektion von Hybridomzellen aus einer Vielzahl von
        Hybridomzellen mittels eines BirA-Expressionsvektors

<130>  NEM185-04

<160>  18

<170>  PatentIn version 3.5

<210>  1
<211>  15
<212>  PRT
<213>  AviTag Artificial

<400>  1

Gly Leu Asn Asp Ile Phe Glu Ala Gln Lys Ile Glu Trp His Glu
1               5                   10                  15


<210>  2
<211>  13
<212>  PRT
<213>  AviTag Artificial

<400>  2

Leu Asn Asp Ile Phe Glu Ala Gln Lys Ile Glu Trp His
1               5                   10


<210>  3
<211>  13
<212>  PRT
<213>  Artificial

<220>
<223>  Biotinylation


<220>
<221>  MISC_FEATURE
<222>  (2)..(2)
<223>  Xaa can be N, C or any amino acid or be deleted

<220>
<221>  MISC_FEATURE
<222>  (3)..(3)
<223>  Xaa can be any amino acid except L, V, I, W, F, Y

<220>
<221>  MISC_FEATURE
<222>  (5)..(5)
<223>  Xaa can be F or L

<220>
<221>  MISC_FEATURE
<222>  (6)..(6)
<223>  Xaa can be E or D

<220>
<221>  MISC_FEATURE
<222>  (7)..(7)
<223>  Xaa can be A, G, S, T

<220>
<221>  MISC_FEATURE
<222>  (8)..(8)
<223>  Xaa can be Q or M

<220>
<221>  MISC_FEATURE
<222>  (10)..(10)
<223>  Xaa can be I, M, V

<220>
<221>  MISC_FEATURE
<222>  (11)..(11)
<223>  Xaa can be E, L, V, Y, I

<220>
<221>  MISC_FEATURE
<222>  (12)..(12)
<223>  Xaa can be W,Y,V,F,L, I

<220>
<221>  MISC_FEATURE
<222>  (13)..(13)
<223>  Xaa can be R,H or any amino acid except D, E

<400>  3

Leu Xaa Xaa Ile Xaa Xaa Xaa Xaa Lys Xaa Xaa Xaa Xaa
1               5                   10


<210>  4
<211>  330
<212>  PRT
<213>  Escherichia coli

<400>  4

Met Lys Asp Asn Thr Val Pro Leu Lys Leu Ile Ala Leu Leu Ala Asn
1               5                   10                  15


Gly Glu Phe His Ser Gly Glu Gln Leu Gly Glu Thr Leu Gly Met Ser
            20                  25                  30


Arg Ala Ala Ile Asn Lys His Ile Gln Thr Leu Arg Asp Trp Gly Val
        35                  40                  45


Asp Val Phe Thr Val Pro Gly Lys Gly Tyr Ser Leu Pro Glu Pro Ile
    50                  55                  60


Gln Leu Leu Asn Ala Lys Gln Ile Leu Gly Gln Leu Asp Gly Gly Ser
65                  70                  75                  80

Val Ala Val Leu Pro Val Ile Asp Ser Thr Asn Gln Tyr Leu Leu Asp
                85                  90                  95

Arg Ile Gly Glu Leu Lys Ser Gly Asp Ala Cys Ile Ala Glu Tyr Gln
            100                 105                 110

Gln Ala Gly Arg Gly Arg Arg Gly Arg Lys Trp Phe Ser Pro Phe Gly
        115                 120                 125

Ala Asn Leu Tyr Leu Ser Met Phe Trp Arg Leu Glu Gln Gly Pro Ala
    130                 135                 140

Ala Ala Ile Gly Leu Ser Leu Val Ile Gly Ile Val Met Ala Glu Val
145                 150                 155                 160

Leu Arg Lys Leu Gly Ala Asp Lys Val Arg Val Lys Trp Pro Asn Asp
                165                 170                 175

Leu Tyr Leu Gln Asp Arg Lys Leu Ala Gly Ile Leu Val Glu Leu Thr
            180                 185                 190

Gly Lys Thr Gly Asp Ala Ala Gln Ile Val Ile Gly Ala Gly Ile Asn
        195                 200                 205

Met Ala Met Arg Arg Val Glu Glu Ser Val Val Asn Gln Gly Trp Ile
    210                 215                 220

Thr Leu Gln Glu Ala Gly Ile Asn Leu Asp Arg Asn Thr Leu Ala Ala
225                 230                 235                 240

Met Leu Ile Arg Glu Leu Arg Ala Ala Leu Glu Leu Phe Glu Gln Glu
                245                 250                 255

Gly Leu Ala Pro Tyr Leu Ser Arg Trp Glu Lys Leu Asp Asn Phe Ile
            260                 265                 270

Asn Arg Pro Val Lys Leu Ile Ile Gly Asp Lys Glu Ile Phe Gly Ile
        275                 280                 285

Ser Arg Gly Ile Asp Lys Gln Gly Ala Leu Leu Leu Glu Gln Asp Gly
    290                 295                 300

Ile Ile Lys Pro Trp Met Gly Gly Glu Ile Ser Leu Arg Ser Ala Glu
305                 310                 315                 320

Lys Gly Ser Thr Ser Gly Ser Gly Gly Ser
                325                 330

<210>  5
<211>  27
<212>  DNA
<213>  HA-Tag artificial

<400>  5
tatccatatg atgttccaga ttatgct                                          27


<210>  6
<211>  9
<212>  PRT
<213>  HA-Tag artificial

<400>  6

Tyr Pro Tyr Asp Val Pro Asp Tyr Ala
1               5


<210>  7
<211>  45
<212>  DNA
<213>  AviTag artificial

<400>  7
ggcctgaacg acatcttcga ggcccagaag atcgagtggc acgag                      45


<210>  8
<211>  72
<212>  DNA
<213>  Human (EGFR Signal)

<400>  8
atgcgaccct ccgggacggc cggggcagcg ctcctggcgc tgctggctgc gctctgcccg      60

gcgagtcggg ct                                                          72


<210>  9
<211>  24
<212>  PRT
<213>  Human (EGFR-Signal)

<400>  9

Met Arg Pro Ser Gly Thr Ala Gly Ala Ala Leu Leu Ala Leu Leu Ala
1               5                   10                  15


Ala Leu Cys Pro Ala Ser Arg Ala
            20


<210>  10
<211>  1956
<212>  DNA
<213>  Human (EGFR)

<400>  10

ctggaggaaa agaaagtttg ccaaggcacg agtaacaagc tcacgcagtt gggcactttt 60

gaagatcatt ttctcagcct ccagaggatg ttcaataact gtgaggtggt ccttgggaat 120

ttggaaatta cctatgtgca gaggaattat gatctttcct tcttaaagac catccaggag 180

gtggctggtt atgccctcat tgccctcaac acagtggagc gaattccttt ggaaaacctg 240

cagatcatca gaggaaatat gtactacgaa aattcctatg ccttagcagt cttatctaac 300

tatgatgcaa ataaaaccgg actgaaggag ctgcccatga gaaatttaca ggaaatcctg 360

catggcgccg tgcggttcag caacaaccct gccctgtgca acgtggagag catccagtgg 420

cgggacatag tcagcagtga ctttctcagc aacatgtcga tggacttcca gaaccacctg 480

ggcagctgcc aaaagtgtga tccaagctgt cccaatggga gctgctgggg tgcaggagag 540

gagaactgcc agagactgac caaaatcatc tgtgcccagc agtgctccgg gcgctgccgt 600

ggcaagtccc ccagtgactg ctgccacaac cagtgtgctg caggctgcac aggcccccgg 660

gagagcgact gcctggtctg ccgcaaattc cgagacgaag ccacgtgcaa ggacacctgc 720

cccccactca tgctctacaa ccccaccacg taccagatgg atgtgaaccc cgagggcaaa 780

tacagctttg gtgccacctg cgtgaagaag tgtccccgta attatgtggt gacagatcac 840

ggctcgtgcg tccgagcctg tggggccgac agctatgaga tggaggaaga cggcgtccgc 900

aagtgtaaga agtgcgaagg gccttgccgc aaagtgtgta acggaatagg tattggtgaa 960

tttaaagact cactctccat aaatgctacg aatattaaac acttcaaaaa ctgcacctcc 1020

atcagtggcg atctccacat cctgccggtg gcatttaggg gtgactcctt cacacatact 1080

cctcctctgg atccacagga actggatatt ctgaaaaccg taaaggaaat cacagggttt 1140

ttgctgattc aggcttggcc tgaaaacagg acggacctcc atgcctttga gaacctagaa 1200

atcatacgcg gcaggaccaa gcaacatggt cagttttctc ttgcagtcgt cagcctgaac 1260

ataacatcct tgggattacg ctccctcaag gagataagtg atggagatgt gataatttca 1320

ggaaacaaaa atttgtgcta tgcaaataca ataaactgga aaaaactgtt tgggacctcc 1380

ggtcagaaaa ccaaaattat aagcaacaga ggtgaaaaca gctgcaaggc cacaggccag 1440

gtctgccatg ccttgtgctc ccccgagggc tgctggggcc cggagcccag ggactgcgtc 1500

tcttgccgga atgtcagccg aggcagggaa tgcgtggaca agtgcaacct tctggagggt 1560

gagccaaggg agtttgtgga gaactctgag tgcatacagt gccacccaga gtgcctgcct 1620

caggccatga acatcacctg cacaggacgg ggaccagaca actgtatcca gtgtgcccac 1680

tacattgacg gcccccactg cgtcaagacc tgcccggcag gagtcatggg agaaaacaac 1740

accctggtct ggaagtacgc agacgccggc catgtgtgcc acctgtgcca tccaaactgc 1800

acctacggat gcactgggcc aggtcttgaa ggctgtccaa cgaatgggcc taagatcccg 1860

tccatcgcca ctgggatggt gggggccctc ctcttgctgc tggtggtggc cctggggatc 1920

ggcctcttca tgcgaaggcg ccacatcgtt cggtaa                                    1956

<210>  11
<211>  651
<212>  PRT
<213>  Human (EGFR)

<400>  11

Leu Glu Glu Lys Lys Val Cys Gln Gly Thr Ser Asn Lys Leu Thr Gln
1               5                   10                  15

Leu Gly Thr Phe Glu Asp His Phe Leu Ser Leu Gln Arg Met Phe Asn
            20                  25                  30

Asn Cys Glu Val Val Leu Gly Asn Leu Glu Ile Thr Tyr Val Gln Arg
        35                  40                  45

Asn Tyr Asp Leu Ser Phe Leu Lys Thr Ile Gln Glu Val Ala Gly Tyr
    50                  55                  60

Ala Leu Ile Ala Leu Asn Thr Val Glu Arg Ile Pro Leu Glu Asn Leu
65                  70                  75                  80

Gln Ile Ile Arg Gly Asn Met Tyr Tyr Glu Asn Ser Tyr Ala Leu Ala
                85                  90                  95

Val Leu Ser Asn Tyr Asp Ala Asn Lys Thr Gly Leu Lys Glu Leu Pro
            100                 105                 110

Met Arg Asn Leu Gln Glu Ile Leu His Gly Ala Val Arg Phe Ser Asn
        115                 120                 125

Asn Pro Ala Leu Cys Asn Val Glu Ser Ile Gln Trp Arg Asp Ile Val
    130                 135                 140

Ser Ser Asp Phe Leu Ser Asn Met Ser Met Asp Phe Gln Asn His Leu
145                 150                 155                 160

Gly Ser Cys Gln Lys Cys Asp Pro Ser Cys Pro Asn Gly Ser Cys Trp
                165                 170                 175

Gly Ala Gly Glu Glu Asn Cys Gln Arg Leu Thr Lys Ile Ile Cys Ala
            180                 185                 190

Gln Gln Cys Ser Gly Arg Cys Arg Gly Lys Ser Pro Ser Asp Cys Cys
        195                 200                 205

His Asn Gln Cys Ala Ala Gly Cys Thr Gly Pro Arg Glu Ser Asp Cys
    210                 215                 220

Leu Val Cys Arg Lys Phe Arg Asp Glu Ala Thr Cys Lys Asp Thr Cys
225                 230                 235                 240

Pro Pro Leu Met Leu Tyr Asn Pro Thr Thr Tyr Gln Met Asp Val Asn
                245                 250                 255

Pro Glu Gly Lys Tyr Ser Phe Gly Ala Thr Cys Val Lys Lys Cys Pro
            260                 265                 270

Arg Asn Tyr Val Val Thr Asp His Gly Ser Cys Val Arg Ala Cys Gly
        275                 280                 285

Ala Asp Ser Tyr Glu Met Glu Glu Asp Gly Val Arg Lys Cys Lys Lys
    290                 295                 300

Cys Glu Gly Pro Cys Arg Lys Val Cys Asn Gly Ile Gly Ile Gly Glu
305                 310                 315                 320

Phe Lys Asp Ser Leu Ser Ile Asn Ala Thr Asn Ile Lys His Phe Lys
                325                 330                 335

Asn Cys Thr Ser Ile Ser Gly Asp Leu His Ile Leu Pro Val Ala Phe
            340                 345                 350

Arg Gly Asp Ser Phe Thr His Thr Pro Pro Leu Asp Pro Gln Glu Leu
        355                 360                 365

Asp Ile Leu Lys Thr Val Lys Glu Ile Thr Gly Phe Leu Leu Ile Gln
    370                 375                 380

Ala Trp Pro Glu Asn Arg Thr Asp Leu His Ala Phe Glu Asn Leu Glu
385                 390                 395                 400

Ile Ile Arg Gly Arg Thr Lys Gln His Gly Gln Phe Ser Leu Ala Val
                405                 410                 415

Val Ser Leu Asn Ile Thr Ser Leu Gly Leu Arg Ser Leu Lys Glu Ile
            420                 425                 430

Ser Asp Gly Asp Val Ile Ile Ser Gly Asn Lys Asn Leu Cys Tyr Ala
        435                 440                 445

Asn Thr Ile Asn Trp Lys Lys Leu Phe Gly Thr Ser Gly Gln Lys Thr
    450                 455                 460

Lys Ile Ile Ser Asn Arg Gly Glu Asn Ser Cys Lys Ala Thr Gly Gln
465                 470                 475                 480

Val Cys His Ala Leu Cys Ser Pro Glu Gly Cys Trp Gly Pro Glu Pro
                485                 490                 495

Arg Asp Cys Val Ser Cys Arg Asn Val Ser Arg Gly Arg Glu Cys Val
            500                 505                 510

Asp Lys Cys Asn Leu Leu Glu Gly Glu Pro Arg Glu Phe Val Glu Asn
        515                 520                 525

Ser Glu Cys Ile Gln Cys His Pro Glu Cys Leu Pro Gln Ala Met Asn
    530                 535                 540

Ile Thr Cys Thr Gly Arg Gly Pro Asp Asn Cys Ile Gln Cys Ala His
545                 550                 555                 560

Tyr Ile Asp Gly Pro His Cys Val Lys Thr Cys Pro Ala Gly Val Met
                565                 570                 575

Gly Glu Asn Asn Thr Leu Val Trp Lys Tyr Ala Asp Ala Gly His Val
            580                 585                 590

Cys His Leu Cys His Pro Asn Cys Thr Tyr Gly Cys Thr Gly Pro Gly
        595                 600                 605

Leu Glu Gly Cys Pro Thr Asn Gly Pro Lys Ile Pro Ser Ile Ala Thr
    610                 615                 620

Gly Met Val Gly Ala Leu Leu Leu Leu Leu Val Val Ala Leu Gly Ile
625                 630                 635                 640

Gly Leu Phe Met Arg Arg Arg His Ile Val Arg
                645                 650

<210>  12
<211>  552
<212>  DNA
<213>  IRES artificial

<400>  12
acgttactgg ccgaagccgc ttggaataag gccggtgtgc gtttgtctat atgttatttt      60

ccaccatatt gccgtctttt ggcaatgtga gggcccggaa acctggccct gtcttcttga     120

cgagcattcc taggggtctt tcccctctcg ccaaaggaat gcaaggtctg ttgaatgtcg     180

tgaaggaagc agttcctctg gaagcttctt gaagacaaac aacgtctgta gcgacccttt     240

gcaggcagcg gaacccccca cctggcgaca ggtgcctctg cggccaaaag ccacgtgtat 300

aagatacacc tgcaaaggcg gcacaacccc agtgccacgt tgtgagttgg atagttgtgg 360

aaagagtcaa atggctctcc tcaagcgtat tcaacaaggg gctgaaggat gcccagaagg 420

taccccattg tatgggatct gatctggggc ctcggtgcac atgctttaca tgtgtttagt 480

cgaggttaaa aaaacgtcta ggccccccga accacgggga cgtggttttc ctttgaaaaa 540

cacgatgata at 552

<210> 13
<211> 1005
<212> DNA
<213> Escherichia coli

<400> 13
atgaaggata acaccgtgcc actgaaattg attgccctgt tagcgaacgg tgaatttcac 60

tctggcgagc agttgggtga aacgctggga atgagccggg cggctattaa taaacacatt 120

cagacactgc gtgactgggg cgttgatgtc tttaccgttc cgggtaaagg atacagcctg 180

cctgagccta tccagttact taatgctaaa cagatattgg gtcagctgga tggcggtagt 240

gtagccgtgc tgcctgtgat tgactccacg aatcagtacc ttcttgatcg tatcggagag 300

cttaaatcgg gcgatgcttg cattgcagaa taccagcagg ctggccgtgg tcgccggggt 360

cggaaatggt tttcgccttt tggcgcaaac ttatatttgt cgatgttctg gcgtctggaa 420

caaggcccgg cggcggcgat tggtttaagt ctggttatcg gtatcgtgat ggcggaagta 480

ttacgcaagc tgggtgcaga taaagttcgt gttaaatggc ctaatgacct ctatctgcag 540

gatcgcaagc tggcaggcat tctggtggag ctgactggca aaactggcga tgcggcgcaa 600

atagtcattg gagccgggat caacatggca atgcgccgtg ttgaagagag tgtcgttaat 660

caggggtgga tcacgctgca ggaagcgggg atcaatctcg atcgtaatac gttggcggcc 720

atgctaatac gtgaattacg tgctgcgttg gaactcttcg aacaagaagg attggcacct 780

tatctgtcgc gctgggaaaa gctggataat tttattaatc gcccagtgaa acttatcatt 840

ggtgataaag aaatatttgg catttcacgc ggaatagaca aacagggggc tttattactt 900

gagcaggatg gaataataaa accctggatg ggcggtgaaa tatccctgcg tagtgcagaa 960

aaagggtcta catctggatc tgggggttca aaggacgagc tgtga 1005

<210> 14
<211> 334
<212> PRT
<213> Escherichia coli

<400> 14

Met Lys Asp Asn Thr Val Pro Leu Lys Leu Ile Ala Leu Leu Ala Asn
1               5                   10                  15

Gly Glu Phe His Ser Gly Glu Gln Leu Gly Glu Thr Leu Gly Met Ser
            20                  25                  30

Arg Ala Ala Ile Asn Lys His Ile Gln Thr Leu Arg Asp Trp Gly Val
        35                  40                  45

Asp Val Phe Thr Val Pro Gly Lys Gly Tyr Ser Leu Pro Glu Pro Ile
    50                  55                  60

Gln Leu Leu Asn Ala Lys Gln Ile Leu Gly Gln Leu Asp Gly Gly Ser
65                  70                  75                  80

Val Ala Val Leu Pro Val Ile Asp Ser Thr Asn Gln Tyr Leu Leu Asp
                85                  90                  95

Arg Ile Gly Glu Leu Lys Ser Gly Asp Ala Cys Ile Ala Glu Tyr Gln
            100                 105                 110

Gln Ala Gly Arg Gly Arg Arg Gly Arg Lys Trp Phe Ser Pro Phe Gly
        115                 120                 125

Ala Asn Leu Tyr Leu Ser Met Phe Trp Arg Leu Glu Gln Gly Pro Ala
    130                 135                 140

Ala Ala Ile Gly Leu Ser Leu Val Ile Gly Ile Val Met Ala Glu Val
145                 150                 155                 160

Leu Arg Lys Leu Gly Ala Asp Lys Val Arg Val Lys Trp Pro Asn Asp
                165                 170                 175

Leu Tyr Leu Gln Asp Arg Lys Leu Ala Gly Ile Leu Val Glu Leu Thr
            180                 185                 190

Gly Lys Thr Gly Asp Ala Ala Gln Ile Val Ile Gly Ala Gly Ile Asn
        195                 200                 205

Met Ala Met Arg Arg Val Glu Glu Ser Val Val Asn Gln Gly Trp Ile
    210                 215                 220

Thr Leu Gln Glu Ala Gly Ile Asn Leu Asp Arg Asn Thr Leu Ala Ala
225                 230                 235                 240

Met Leu Ile Arg Glu Leu Arg Ala Ala Leu Glu Leu Phe Glu Gln Glu
                245                 250                 255

Gly Leu Ala Pro Tyr Leu Ser Arg Trp Glu Lys Leu Asp Asn Phe Ile

260                 265                 270

Asn Arg Pro Val Lys Leu Ile Ile Gly Asp Lys Glu Ile Phe Gly Ile
        275                 280                 285

Ser Arg Gly Ile Asp Lys Gln Gly Ala Leu Leu Leu Glu Gln Asp Gly
    290                 295                 300

Ile Ile Lys Pro Trp Met Gly Gly Glu Ile Ser Leu Arg Ser Ala Glu
305                 310                 315                 320

Lys Gly Ser Thr Ser Gly Ser Gly Gly Ser Lys Asp Glu Leu
                325                 330

<210>  15
<211>  3895
<212>  DNA
<213>  Artificial

<220>
<223>  Expressionsvektor

<400>  15
atgcgaccct ccgggacggc cggggcagcg ctcctggcgc tgctggctgc gctctgcccg      60

gcgagtcggg ctctggagga aggctcgtct aagcttggct cgtctgggta tccatatgat     120

gttccagatt atgctggggc ccagccggcc agatctggcg gcggcctgaa cgacatcttc     180

gaggcccaga agatcgagtg gcacgagggc gcgccggcta ccggaagcag cgggctggag     240

gaaaagaaag tttgccaagg cacgagtaac aagctcacgc agttgggcac ttttgaagat     300

cattttctca gcctccagag gatgttcaat aactgtgagg tggtccttgg gaatttggaa     360

attacctatg tgcagaggaa ttatgatctt tccttcttaa agaccatcca ggaggtggct     420

ggttatgccc tcattgccct caacacagtg gagcgaattc ctttggaaaa cctgcagatc     480

atcagaggaa atatgtacta cgaaaattcc tatgccttag cagtcttatc taactatgat     540

gcaaataaaa ccggactgaa ggagctgccc atgagaaatt tacaggaaat cctgcatggc     600

gccgtgcggt tcagcaacaa ccctgccctg tgcaacgtgg agagcatcca gtggcgggac     660

atagtcagca gtgactttct cagcaacatg tcgatggact tccagaacca cctgggcagc     720

tgccaaaagt gtgatccaag ctgtcccaat gggagctgct ggggtgcagg agaggagaac     780

tgccagagac tgaccaaaat catctgtgcc cagcagtgct ccgggcgctg ccgtggcaag     840

tcccccagtg actgctgcca caaccagtgt gctgcaggct gcacaggccc ccgggagagc     900

gactgcctgg tctgccgcaa attccgagac gaagccacgt gcaaggacac ctgcccccca     960

ctcatgctct acaaccccac cacgtaccag atggatgtga accccgaggg caaatacagc    1020

tttggtgcca cctgcgtgaa gaagtgtccc cgtaattatg tggtgacaga tcacggctcg    1080

tgcgtccgag cctgtggggc cgacagctat gagatggagg aagacggcgt ccgcaagtgt 1140

aagaagtgcg aagggccttg ccgcaaagtg tgtaacggaa taggtattgg tgaatttaaa 1200

gactcactct ccataaatgc tacgaatatt aaacacttca aaaactgcac ctccatcagt 1260

ggcgatctcc acatcctgcc ggtggcattt aggggtgact ccttcacaca tactcctcct 1320

ctggatccac aggaactgga tattctgaaa accgtaaagg aaatcacagg gtttttgctg 1380

attcaggctt ggcctgaaaa caggacggac ctccatgcct ttgagaacct agaaatcata 1440

cgcggcagga ccaagcaaca tggtcagttt tctcttgcag tcgtcagcct gaacataaca 1500

tccttgggat tacgctccct caaggagata agtgatggag atgtgataat ttcaggaaac 1560

aaaaatttgt gctatgcaaa tacaataaac tggaaaaaac tgtttgggac ctccggtcag 1620

aaaaccaaaa ttataagcaa cagaggtgaa aacagctgca aggccacagg ccaggtctgc 1680

catgccttgt gctcccccga gggctgctgg ggcccggagc ccagggactg cgtctcttgc 1740

cggaatgtca gccgaggcag ggaatgcgtg gacaagtgca accttctgga gggtgagcca 1800

agggagtttg tggagaactc tgagtgcata cagtgccacc cagagtgcct gcctcaggcc 1860

atgaacatca cctgcacagg acggggacca gacaactgta tccagtgtgc ccactacatt 1920

gacggccccc actgcgtcaa gacctgcccg gcaggagtca tgggagaaaa caacaccctg 1980

gtctggaagt acgcagacgc cggccatgtg tgccacctgt gccatccaaa ctgcacctac 2040

ggatgcactg ggccaggtct tgaaggctgt ccaacgaatg ggcctaagat cccgtccatc 2100

gccactggga tggtgggggc cctcctcttg ctgctggtgg tggccctggg gatcggcctc 2160

ttcatgcgaa ggcgccacat cgttcggtaa gcggccgctc tagcccaatt ccgccccccc 2220

ccccctaacg ttactggccg aagccgcttg gaataaggcc ggtgtgcgtt tgtctatatg 2280

ttattttcca ccatattgcc gtcttttggc aatgtgaggg cccggaaacc tggccctgtc 2340

ttcttgacga gcattcctag gggtctttcc cctctcgcca aaggaatgca aggtctgttg 2400

aatgtcgtga aggaagcagt tcctctggaa gcttcttgaa gacaaacaac gtctgtagcg 2460

accctttgca ggcagcggaa ccccccacct ggcgacaggt gcctctgcgg ccaaaagcca 2520

cgtgtataag atacacctgc aaaggcggca caaccccagt gccacgttgt gagttggata 2580

gttgtggaaa gagtcaaatg gctctcctca agcgtattca acaaggggct gaaggatgcc 2640

cagaaggtac cccattgtat gggatctgat ctggggcctc ggtgcacatg ctttacatgt 2700

gtttagtcga ggttaaaaaa acgtctaggc cccccgaacc acggggacgt ggttttcctt 2760

tgaaaaacac gatgataata tgggccgacc ctccgggacg gccggggcag cgctcctggc 2820

gctgctggct gcgctctgcc cggcgagtcg ggctctggag gaaggctcgt ctaagcttgg 2880

ctcgtctggg atgaaggata acaccgtgcc actgaaattg attgccctgt tagcgaacgg 2940

tgaatttcac tctggcgagc agttgggtga aacgctggga atgagccggg cggctattaa 3000

taaacacatt cagacactgc gtgactgggg cgttgatgtc tttaccgttc cgggtaaagg 3060

atacagcctg cctgagccta tccagttact taatgctaaa cagatattgg gtcagctgga 3120

tggcggtagt gtagccgtgc tgcctgtgat tgactccacg aatcagtacc ttcttgatcg 3180

tatcggagag cttaaatcgg gcgatgcttg cattgcagaa taccagcagg ctggccgtgg 3240

tcgccggggt cggaaatggt tttcgccttt tggcgcaaac ttatatttgt cgatgttctg 3300

gcgtctggaa caaggcccgg cggcggcgat tggtttaagt ctggttatcg gtatcgtgat 3360

ggcggaagta ttacgcaagc tgggtgcaga taaagttcgt gttaaatggc ctaatgacct 3420

ctatctgcag gatcgcaagc tggcaggcat tctggtggag ctgactggca aaactggcga 3480

tgcggcgcaa atagtcattg gagccgggat caacatggca atgcgccgtg ttgaagagag 3540

tgtcgttaat caggggtgga tcacgctgca ggaagcgggg atcaatctcg atcgtaatac 3600

gttggcggcc atgctaatac gtgaattacg tgctgcgttg gaactcttcg aacaagaagg 3660

attggcacct tatctgtcgc gctgggaaaa gctggataat tttattaatc gcccagtgaa 3720

acttatcatt ggtgataaag aaatatttgg catttcacgc ggaatagaca aacagggggc 3780

tttattactt gagcaggatg gaataataaa accctggatg ggcggtgaaa tatccctgcg 3840

tagtgcagaa aaaggtctca catctggatc tgggggttca aaggacgagc tgtga 3895

<210> 16
<211> 3913
<212> DNA
<213> Artificial

<220>
<223> Expressionsvektor

<400> 16
ggtaccgcca ccatgcgacc ctccgggacg gccggggcag cgctcctggc gctgctggct 60

gcgctctgcc cggcgagtcg ggctctggag gaaggctcgt ctaagcttgg ctcgtctggg 120

tatccatatg atgttccaga ttatgctggg gcccagccgg ccagatctgg cggcggcctg 180

aacgacatct tcgaggccca gaagatcgag tggcacgagg gcgcgccggc taccggaagc 240

agcgggctgg aggaaaagaa agtttgccaa ggcacgagta acaagctcac gcagttgggc 300

acctttgaag atcattttct cagcctccag aggatgttca ataactgtga ggtggtcctt 360

gggaatttgg aaattaccta tgtgcagagg aattatgatc tttccttctt aaagaccatc 420

caggaggtgg ctggttatgc cctcattgcc ctcaacacag tggagcgaat tcctttggaa 480

aacctgcaga tcatcagagg aaatatgtac tacgaaaatt cctatgcctt agcagtctta 540

tctaactatg atgcaaataa aaccggactg aaggagctgc ccatgagaaa tttacaggaa 600

atcctgcatg gcgccgtgcg gttcagcaac aaccctgccc tgtgcaacgt ggagagcatc 660

cagtggcggg acatagtcag cagtgacttt ctcagcaaca tgtcgatgga cttccagaac 720

cacctgggca gctgccaaaa gtgtgatcca agctgtccca atgggagctg ctggggtgca 780

ggagaggaga actgccagag actgaccaaa atcatctgtg cccagcagtg ctccgggcgc 840

tgccgtggca agtcccccag tgactgctgc cacaaccagt gtgctgcagg ctgcacaggc 900

ccccgggaga gcgactgcct ggtctgccgc aaattccgag acgaagccac gtgcaaggac 960

acctgccccc cactcatgct ctacaacccc accacgtacc agatggatgt gaaccccgag 1020

ggcaaataca gctttggtgc cacctgcgtg aagaagtgtc cccgtaatta tgtggtgaca 1080

gatcacggct cgtgcgtccg agcctgtggg gccgacagct atgagatgga ggaagacggc 1140

gtccgcaagt gtaagaagtg cgaagggcct tgccgcaaag tgtgtaacgg aataggtatt 1200

ggtgaattta aagactcact ctccataaat gctacgaata ttaaacactt caaaaactgc 1260

acctccatca gtggcgatct ccacatcctg ccggtggcat ttaggggtga ctccttcaca 1320

catactcctc ctctggatcc acaggaactg gatattctga aaaccgtaaa ggaaatcaca 1380

gggtttttgc tgattcaggc ttggcctgaa aacaggacgg acctccatgc ctttgagaac 1440

ctagaaatca tacgcggcag gaccaagcaa catggtcagt tttctcttgc agtcgtcagc 1500

ctgaacataa catccttggg attacgctcc ctcaaggaga taagtgatgg agatgtgata 1560

atttcaggaa acaaaaattt gtgctatgca aatacaataa actggaaaaa actgtttggg 1620

acctccggtc agaaaaccaa aattataagc aacagaggtg aaaacagctg caaggccaca 1680

ggccaggtct gccatgcctt gtgctccccc gagggctgct ggggcccgga gcccagggac 1740

tgcgtctctt gccggaatgt cagccgaggc agggaatgcg tggacaagtg caaccttctg 1800

gagggtgagc caagggagtt tgtggagaac tctgagtgca tacagtgcca cccagagtgc 1860

ctgcctcagg ccatgaacat cacctgcaca ggacggggac cagacaactg tatccagtgt 1920

gcccactaca ttgacggccc ccactgcgtc aagacctgcc cggcaggagt catgggagaa 1980

aacaacaccc tggtctggaa gtacgcagac gccggccatg tgtgccacct gtgccatcca 2040

aactgcacct acggatgcac tgggccaggt cttgaaggct gtccaacgaa tgggcctaag 2100

atcccgtcca tcgccactgg gatggtgggg gccctcctct tgctgctggt ggtggccctg 2160

gggatcggcc tcttcatgcg aaggcgccac atcgttcggt aagcggccgc tctagcccaa 2220

ttccgccccc ccccccctaa cgttactggc cgaagccgct tggaataagg ccggtgtgcg 2280

tttgtctata tgttattttc caccatattg ccgtcttttg gcaatgtgag ggcccggaaa 2340

cctggccctg tcttcttgac gagcattcct aggggtcttt cccctctcgc caaaggaatg 2400

caaggtctgt tgaatgtcgt gaaggaagca gttcctctgg aagcttcttg aagacaaaca 2460

acgtctgtag cgaccctttg caggcagcgg aaccccccac ctggcgacag gtgcctctgc 2520

ggccaaaagc cacgtgtata agatacacct gcaaaggcgg cacaacccca gtgccacgtt 2580

gtgagttgga tagttgtgga aagagtcaaa tggctctcct caagcgtatt caacaagggg 2640

ctgaaggatg cccagaaggt accccattgt atgggatctg atctggggcc tcggtgcaca 2700

tgctttacat gtgtttagtc gaggttaaaa aaacgtctag gccccccgaa ccacggggac 2760

gtggttttcc tttgaaaaac acgatgataa tatgggccga ccctccggga cggccggggc 2820

agcgctcctg gcgctgctgg ctgcgctctg cccggcgagt cgggctctgg aggaaggctc 2880

gtctaagctt ggctcgtctg ggatgaagga taacaccgtg ccactgaaat tgattgccct 2940

gttagcgaac ggtgaatttc actctggcga gcagttgggt gaaacgctgg gaatgagccg 3000

ggcggctatt aataaacaca ttcagacact gcgtgactgg ggcgttgatg tctttaccgt 3060

tccgggtaaa ggatacagcc tgcctgagcc tatccagtta cttaatgcta aacagatatt 3120

gggtcagctg gatggcggta gtgtagccgt gctgcctgtg attgactcca cgaatcagta 3180

ccttcttgat cgtatcggag agcttaaatc gggcgatgct tgcattgcag aataccagca 3240

ggctggccgt ggtcgccggg gtcggaaatg gttttcgcct tttggcgcaa acttatattt 3300

gtcgatgttc tggcgtctgg aacaaggccc ggcggcggcg attggtttaa gtctggttat 3360

cggtatcgtg atggcggaag tattacgcaa gctgggtgca gataaagttc gtgttaaatg 3420

gcctaatgac ctctatctgc aggatcgcaa gctggcaggc attctggtgg agctgactgg 3480

caaaactggc gatgcggcgc aaatagtcat tggagccggg atcaacatgg caatgcgccg 3540

tgttgaagag agtgtcgtta atcaggggtg gatcacgctg caggaagcgg ggatcaatct 3600

cgatcgtaat acgttggcgg ccatgctaat acgtgaatta cgtgctgcgt tggaactctt 3660

cgaacaagaa ggattggcac cttatctgtc gcgctgggaa aagctggata attttattaa 3720

tcgcccagtg aaacttatca ttggtgataa agaaatattt ggcatttcac gcggaataga 3780

caaacagggg gctttattac ttgagcagga tggaataata aaaccctgga tgggcggtga 3840

aatatccctg cgtagtgcag aaaaagggtc tacatctgga tctgggggtt caaaggacga 3900

gctgtgactc gag 3913

<210> 17
<211> 1274
<212> PRT
<213> Artificial

<220>
<223> Expressionsprodukt

<400> 17

Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Gly Ser Ser Lys Leu
            20                  25                  30
Gly Ser Ser Gly Tyr Pro Tyr Asp Val Pro Asp Tyr Ala Gly Ala Gln
        35                  40                  45
Pro Ala Arg Ser Gly Gly Gly Leu Asn Asp Ile Phe Glu Ala Gln Lys
    50                  55                  60
Ile Glu Trp His Glu Gly Ala Pro Ala Thr Gly Ser Ser Gly Leu Glu
65                  70                  75                  80
Glu Lys Lys Val Cys Gln Gly Thr Ser Asn Lys Leu Thr Gln Leu Gly
                85                  90                  95
Thr Phe Glu Asp His Phe Leu Ser Leu Gln Arg Met Phe Asn Asn Cys
            100                 105                 110
Glu Val Val Leu Gly Asn Leu Glu Ile Thr Tyr Val Gln Arg Asn Tyr
        115                 120                 125
Asp Leu Ser Phe Leu Lys Thr Ile Gln Glu Val Ala Gly Tyr Ala Leu
    130                 135                 140
Ile Ala Leu Asn Thr Val Glu Arg Ile Pro Leu Glu Asn Leu Gln Ile
145                 150                 155                 160
Ile Arg Gly Asn Met Tyr Tyr Glu Asn Ser Tyr Ala Leu Ala Val Leu
                165                 170                 175
Ser Asn Tyr Asp Ala Asn Lys Thr Gly Leu Lys Glu Leu Pro Met Arg
            180                 185                 190
Asn Leu Gln Glu Ile Leu His Gly Ala Val Arg Phe Ser Asn Asn Pro
        195                 200                 205
Ala Leu Cys Asn Val Glu Ser Ile Gln Trp Arg Asp Ile Val Ser Ser
    210                 215                 220
Asp Phe Leu Ser Asn Met Ser Met Asp Phe Gln Asn His Leu Gly Ser
225                 230                 235                 240
Cys Gln Lys Cys Asp Pro Ser Cys Pro Asn Gly Ser Cys Trp Gly Ala
                245                 250                 255
Gly Glu Glu Asn Cys Gln Arg Leu Thr Lys Ile Ile Cys Ala Gln Gln
            260                 265                 270

Cys Ser Gly Arg Cys Arg Gly Lys Ser Pro Ser Asp Cys Cys His Asn
        275                 280                 285

Gln Cys Ala Ala Gly Cys Thr Gly Pro Arg Glu Ser Asp Cys Leu Val
    290                 295                 300

Cys Arg Lys Phe Arg Asp Glu Ala Thr Cys Lys Asp Thr Cys Pro Pro
305                 310                 315                 320

Leu Met Leu Tyr Asn Pro Thr Thr Tyr Gln Met Asp Val Asn Pro Glu
                325                 330                 335

Gly Lys Tyr Ser Phe Gly Ala Thr Cys Val Lys Lys Cys Pro Arg Asn
            340                 345                 350

Tyr Val Val Thr Asp His Gly Ser Cys Val Arg Ala Cys Gly Ala Asp
        355                 360                 365

Ser Tyr Glu Met Glu Glu Asp Gly Val Arg Lys Cys Lys Lys Cys Glu
    370                 375                 380

Gly Pro Cys Arg Lys Val Cys Asn Gly Ile Gly Ile Gly Glu Phe Lys
385                 390                 395                 400

Asp Ser Leu Ser Ile Asn Ala Thr Asn Ile Lys His Phe Lys Asn Cys
                405                 410                 415

Thr Ser Ile Ser Gly Asp Leu His Ile Leu Pro Val Ala Phe Arg Gly
            420                 425                 430

Asp Ser Phe Thr His Thr Pro Pro Leu Asp Pro Gln Glu Leu Asp Ile
        435                 440                 445

Leu Lys Thr Val Lys Glu Ile Thr Gly Phe Leu Leu Ile Gln Ala Trp
    450                 455                 460

Pro Glu Asn Arg Thr Asp Leu His Ala Phe Glu Asn Leu Glu Ile Ile
465                 470                 475                 480

Arg Gly Arg Thr Lys Gln His Gly Gln Phe Ser Leu Ala Val Val Ser
                485                 490                 495

Leu Asn Ile Thr Ser Leu Gly Leu Arg Ser Leu Lys Glu Ile Ser Asp
            500                 505                 510

Gly Asp Val Ile Ile Ser Gly Asn Lys Asn Leu Cys Tyr Ala Asn Thr
        515                 520                 525

Ile Asn Trp Lys Lys Leu Phe Gly Thr Ser Gly Gln Lys Thr Lys Ile
    530                 535                 540

Ile Ser Asn Arg Gly Glu Asn Ser Cys Lys Ala Thr Gly Gln Val Cys
545                 550                 555                 560

His Ala Leu Cys Ser Pro Glu Gly Cys Trp Gly Pro Glu Pro Arg Asp
                565                 570                 575

Cys Val Ser Cys Arg Asn Val Ser Arg Gly Arg Glu Cys Val Asp Lys
            580                 585                 590

Cys Asn Leu Leu Glu Gly Glu Pro Arg Glu Phe Val Glu Asn Ser Glu
        595                 600                 605

Cys Ile Gln Cys His Pro Glu Cys Leu Pro Gln Ala Met Asn Ile Thr
    610                 615                 620

Cys Thr Gly Arg Gly Pro Asp Asn Cys Ile Gln Cys Ala His Tyr Ile
625                 630                 635                 640

Asp Gly Pro His Cys Val Lys Thr Cys Pro Ala Gly Val Met Gly Glu
                645                 650                 655

Asn Asn Thr Leu Val Trp Lys Tyr Ala Asp Ala Gly His Val Cys His
            660                 665                 670

Leu Cys His Pro Asn Cys Thr Tyr Gly Cys Thr Gly Pro Gly Leu Glu
        675                 680                 685

Gly Cys Pro Thr Asn Gly Pro Lys Ile Pro Ser Ile Ala Thr Gly Met
    690                 695                 700

Val Gly Ala Leu Leu Leu Leu Leu Val Val Ala Leu Gly Ile Gly Leu
705                 710                 715                 720

Phe Met Arg Arg Arg His Ile Val Arg Thr Leu Leu Ala Glu Ala Ala
                725                 730                 735

Trp Asn Lys Ala Gly Val Arg Leu Ser Ile Cys Tyr Phe Pro Pro Tyr
            740                 745                 750

Cys Arg Leu Leu Ala Met Gly Pro Gly Asn Leu Ala Leu Ser Ser Arg
        755                 760                 765

Ala Phe Leu Gly Val Phe Pro Leu Ser Pro Lys Glu Cys Lys Val Cys

770                 775                 780

Met Ser Arg Lys Gln Phe Leu Trp Lys Leu Leu Glu Asp Lys Gln Arg
785                 790                 795                 800

Leu Arg Pro Phe Ala Gly Ser Gly Thr Pro His Leu Ala Thr Gly Ala
                805                 810                 815

Ser Ala Ala Lys Ser His Val Tyr Lys Ile His Leu Gln Arg Arg His
            820                 825                 830

Asn Pro Ser Ala Thr Leu Val Gly Leu Trp Lys Glu Ser Asn Gly Ser
        835                 840                 845

Pro Gln Ala Tyr Ser Thr Arg Gly Arg Met Pro Arg Arg Tyr Pro Ile
    850                 855                 860

Val Trp Asp Leu Ile Trp Gly Leu Gly Ala His Ala Leu His Val Phe
865                 870                 875                 880

Ser Arg Gly Lys Asn Val Ala Pro Arg Thr Thr Gly Thr Trp Phe Ser
                885                 890                 895

Phe Glu Lys His Asp Asp Asn Met Gly Arg Pro Ser Gly Thr Ala Gly
            900                 905                 910

Ala Ala Leu Leu Ala Leu Leu Ala Ala Leu Cys Pro Ala Ser Arg Ala
        915                 920                 925

Leu Glu Glu Gly Ser Ser Lys Leu Gly Ser Ser Gly Met Lys Asp Asn
    930                 935                 940

Thr Val Pro Leu Lys Leu Ile Ala Leu Leu Ala Asn Gly Glu Phe His
945                 950                 955                 960

Ser Gly Glu Gln Leu Gly Glu Thr Leu Gly Met Ser Arg Ala Ala Ile
                965                 970                 975

Asn Lys His Ile Gln Thr Leu Arg Asp Trp Gly Val Asp Val Phe Thr
            980                 985                 990

Val Pro Gly Lys Gly Tyr Ser Leu  Pro Glu Pro Ile Gln  Leu Leu Asn
        995                 1000                1005

Ala Lys  Gln Ile Leu Gly Gln  Leu Asp Gly Gly Ser  Val Ala Val
    1010                1015                1020

Leu Pro Val Ile Asp Ser Thr Asn Gln Tyr Leu Leu Asp Arg Ile
    1025                1030                1035

Gly Glu Leu Lys Ser Gly Asp Ala Cys Ile Ala Glu Tyr Gln Gln
    1040                1045                1050

Ala Gly Arg Gly Arg Arg Gly Arg Lys Trp Phe Ser Pro Phe Gly
    1055                1060                1065

Ala Asn Leu Tyr Leu Ser Met Phe Trp Arg Leu Glu Gln Gly Pro
    1070                1075                1080

Ala Ala Ala Ile Gly Leu Ser Leu Val Ile Gly Ile Val Met Ala
    1085                1090                1095

Glu Val Leu Arg Lys Leu Gly Ala Asp Lys Val Arg Val Lys Trp
    1100                1105                1110

Pro Asn Asp Leu Tyr Leu Gln Asp Arg Lys Leu Ala Gly Ile Leu
    1115                1120                1125

Val Glu Leu Thr Gly Lys Thr Gly Asp Ala Ala Gln Ile Val Ile
    1130                1135                1140

Gly Ala Gly Ile Asn Met Ala Met Arg Arg Val Glu Glu Ser Val
    1145                1150                1155

Val Asn Gln Gly Trp Ile Thr Leu Gln Glu Ala Gly Ile Asn Leu
    1160                1165                1170

Asp Arg Asn Thr Leu Ala Ala Met Leu Ile Arg Glu Leu Arg Ala
    1175                1180                1185

Ala Leu Glu Leu Phe Glu Gln Glu Gly Leu Ala Pro Tyr Leu Ser
    1190                1195                1200

Arg Trp Glu Lys Leu Asp Asn Phe Ile Asn Arg Pro Val Lys Leu
    1205                1210                1215

Ile Ile Gly Asp Lys Glu Ile Phe Gly Ile Ser Arg Gly Ile Asp
    1220                1225                1230

Lys Gln Gly Ala Leu Leu Leu Glu Gln Asp Gly Ile Ile Lys Pro
    1235                1240                1245

Trp Met Gly Gly Glu Ile Ser Leu Arg Ser Ala Glu Lys Gly Ser
    1250                1255                1260

Thr Ser Gly Ser Gly Gly Ser Lys Asp Glu Leu
    1265                1270

<210> 18
<211> 4175
<212> DNA
<213> Artificial

<220>
<223> Expressionsvektor

<400> 18
   taatacgact cactataggg cgaattgggg cgcgccattc tagattaacc ctagaaagat      60
   agtctgcgta aaattgacgc atgcattctt gaaatattgc tctctctttc taaatagcgc     120
   gaatccgtcg ctgtgcattt aggacatctc agtcgccgct tggagctccc gtgaggcgtg     180
   cttgtcaatg cggtaagtgt cactgatttt gaactataac gaccgcgtga gtcaaaatga     240
   cgcatgatta tcttttacgt gacttttaag atttaactca tacgataatt atattgttat     300
   ttcatgttct acttacgtga taacttatta tatatatatt ttcttgttat agatatcaac     360
   tagaatgcta gcagatctcg accgagcttt gcaaagatgg ataaagtttt aaacagagag     420
   gaatctttgc agctaatgga ccttctaggt cttgaaagga gtgggaattg gctccggtgc     480
   ccgtcagtgg gcagagcgca catcgcccac agtccccgag aagttggggg gaggggtcgg     540
   caattgaacc ggtgcctaga gaaggtggcg cggggtaaac tgggaaagtg atgtcgtgta     600
   ctggctccgc ctttttcccg agggtggggg agaaccgtat ataagtgcag tagtcgccgt     660
   gaacgttctt tttcgcaacg ggtttgccgc cagaacacag gtaagtgccg tgtgtggttc     720
   ccgcgggcct ggcctcttta cgggttatgg cccttgcgtg ccttgaatta cttccacctg     780
   gctgcagtac gtgattcttg atcccgagct tcgggttgga agtgggtggg agagttcgag     840
   gccttgcgct taaggagccc cttcgcctcg tgcttgagtt gaggcctggc ctgggcgctg     900
   gggccgccgc gtgcgaatct ggtggcacct tcgcgcctgt ctcgctgctt tcgataagtc     960
   tctagccatt taaaattttt gatgacctgc tgcgacgctt tttttctggc aagatagtct    1020
   tgtaaatgcg ggccaagatc gatctgcaca ctggtatttc ggtttttggg gccgcgggcg    1080
   gcgacggggc ccgtgcgtcc cagcgcacat gttcggcgag gcggggcctg cgagcgcggc    1140
   caccgagaat cggacggggg tagtctcaag ctggccggcc tgctctggtg cctggcctcg    1200
   cgccgccgtg tatcgccccg ccctgggcgg caaggctggc ccggtcggca ccagttgcgt    1260
   gagcggaaag atggccgctt cccggccctg ctgcagggag ctcaaaatgg aggacgcggc    1320
   gctcgggaga gcgggcgggt gagtcaccca cacaaaggaa aagggccttt ccgtcctcag    1380
   ccgtcgcttc atgtgactcc acggagtacc gggcgccgtc caggcacctc gattagttct    1440
   cgatcgagct tttggagtac gtcgtcttta ggttgggggg aggggtttta tgcgatggag    1500
   tttccccaca ctgagtgggt ggagactgaa gttaggccag cttggcactt gatgtaattc 1560
   tccttggaat ttgccctttt tgagtttgga tcttggttca ttctcaagcc tcagacagtg 1620
   gttcaaagtt tttttcttcc atttcaggtg tcgtgaggaa ttagcttgac caagcttcga 1680
   attctgcagt cgacggtacc gccaccatgc gaccctccgg gacggccggg gcagcgctcc 1740
   tggcgctgct ggctgcgctc tgcccggcga gtcgggctct ggaggaaggc tcgtctaagc 1800
   ttggctcgtc tgggtatcca tatgatgttc cagattatgc tggggcccag ccggccagat 1860
   ctggcggcgg cctgaacgac atcttcgagg cccagaagat cgagtggcac gagggcgcgc 1920
   cggctaccgg aagcagcggg ctggaggaaa agaaagtttg ccaaggcacg agtaacaagc 1980
   tcacgcagtt gggcactttt gaagatcatt ttctcagcct ccagaggatg ttcaataact 2040
   gtgaggtggt ccttgggaat ttggaaatta cctatgtgca gaggaattat gatctttcct 2100
   tcttaaagac catccaggag gtggctggtt atgccctcat tgccctcaac acagtggagc 2160
   gaattccttt ggaaaacctg cagatcatca gaggaaatat gtactacgaa aattcctatg 2220
   ccttagcagt cttatctaac tatgatgcaa ataaaaccgg actgaaggag ctgcccatga 2280
   gaaatttaca ggaaatcctg catggcgccg tgcggttcag caacaaccct gccctgtgca 2340
   acgtggagag catccagtgg cgggacatag tcagcagtga ctttctcagc aacatgtcga 2400
   tggacttcca gaaccacctg ggcagctgcc aaaagtgtga tccaagctgt cccaatggga 2460
   gctgctgggg tgcaggagag gagaactgcc agagactgac caaaatcatc tgtgcccagc 2520
   agtgctccgg gcgctgccgt ggcaagtccc ccagtgactg ctgccacaac cagtgtgctg 2580
   caggctgcac aggcccccgg gagagcgact gcctggtctg ccgcaaattc cgagacgaag 2640
   ccacgtgcaa ggacacctgc cccccactca tgctctacaa ccccaccacg taccagatgg 2700
   atgtgaaccc cgagggcaaa tacagctttg gtgccacctg cgtgaagaag tgtccccgta 2760
   attatgtggt gacagatcac ggctcgtgcg tccgagcctg tggggccgac agctatgaga 2820
   tggaggaaga cggcgtccgc aagtgtaaga agtgcgaagg gccttgccgc aaagtgtgta 2880
   acggaatagg tattggtgaa tttaaagact cactctccat aaatgctacg aatattaaac 2940
   acttcaaaaa ctgcacctcc atcagtggcg atctccacat cctgccggtg gcatttaggg 3000
   gtgactcctt cacacatact cctcctctgg atccacagga actggatatt ctgaaaaccg 3060
   taaaggaaat cacagggttt ttgctgattc aggcttggcc tgaaaacagg acggacctcc 3120
   atgcctttga gaacctagaa atcatacgcg gcaggaccaa gcaacatggt cagttttctc 3180
   ttgcagtcgt cagcctgaac ataacatcct tgggattacg ctccctcaag gagataagtg 3240
   atggagatgt gataatttca ggaaacaaaa atttgtgcta tgcaaataca ataaactgga 3300
   aaaaactgtt tgggacctcc ggtcagaaaa ccaaaattat aagcaacaga ggtgaaaaca 3360
   gctgcaaggc cacaggccag gtctgccatg ccttgtgctc ccccgagggc tgctggggcc 3420
   cggagcccag ggactgcgtc tcttgccgga atgtcagccg aggcagggaa tgcgtggaca 3480
   agtgcaacct tctggagggt gagccaaggg agtttgtgga gaactctgag tgcatacagt 3540
   gccacccaga gtgcctgcct caggccatga acatcacctg cacaggacgg ggaccagaca 3600
   actgtatcca gtgtgcccac tacattgacg gcccccactg cgtcaagacc tgcccggcag 3660
   gagtcatggg agaaaacaac accctggtct ggaagtacgc agacgccggc catgtgtgcc 3720
   acctgtgcca tccaaactgc acctacggat gcactgggcc aggtcttgaa ggctgtccaa 3780
   cgaatgggcc taagatcccg tccatcgcca ctgggatggt gggggccctc ctcttgctgc 3840
   tggtggtggc cctggggatc ggcctcttca tgcgaaggcg ccacatcgtt cggtaagcgg 3900
   ccgcctcgag tctagagggc ccgtttaaac ccgctgatca gcctcgactg tgccttctag 3960
   ttgccagcca tctgttgttt gcccctcccc cgtgccttcc ttgaccctgg aaggtgccac 4020
   tcccactgtc ctttcctaat aaaatgagga aattgcatcg cattgtctga gtaggtgtca 4080
   ttctattctg gggggtgggg tggggcagga acagcaaggg ggaggattgg gaagacaata 4140
   gcaggcatgc tggggatgcg gtgggctcta tggct 4175